# EUROPEAN PATENT SPECIFICATION

(11) **EP 2 654 863 B1**
(45) Date of publication and mention of the grant of the patent: **21.09.2016**
(21) Application number: 11808699.0
(22) Date of filing: 23.12.2011
(51) Int. Cl.: A61M 35/00, A61M 11/06

(54) **POWDER DELIVERY DEVICE**
PULVERFREISETZUNGSVORRICHTUNG
DISPOSITIF DE DISTRIBUTION DE POUDRE

(30) Priority: 23.12.2010 GB 201021881
(43) Date of publication of application: 30.10.2013
(73) Proprietor: ProFibrix BV, 2333 CK Leiden (NL)
(72) Inventor: GREENHALGH, Paul, Cambridge, Cambridgeshire CB10 1SX (GB); SCHUTTE, Eliane, NL-2333 CK Leiden (NL)
(74) Representative: Jones, Nicholas Andrew
(86) International application number: PCT/GB2011/052586
(87) International publication number: WO 2012/085600

(56) References cited:
- WO-A2-2005/037443
- WO-A2-2010/070333
- DE-C- 619 625
- FR-A1- 2 863 503
- US-A- 6 024 129
- US-A1- 2011 230 820

## Description

This invention relates to improvements in a device for the dispensing of powder. In particular, the invention relates to an improvement in devices of the general type disclosed in International Patent Application WO2010/070333. Such devices are of particular utility in surgical procedures or other medical applications, for the topical delivery of powder to an internal or external surface of the body.

In devices of the type disclosed in WO010/070333, a flow of gas entrains powder that is to be dispensed and carries that powder out of the device via a barrel. The barrel serves to direct the powder to the desired application site.

In the devices illustrated in WO010/070333, the barrels are rather short and are of simple construction.

It has now surprisingly been found that the performance of devices of the type disclosed in WO010/070333 may be substantially improved by modifications to the design of the barrel. In particular, elongation of the barrel and the incorporation of certain structural features in the internal bore of the barrel may lead to an improvements. FR 2863503 already discloses an elongated barrel.

Thus, according to the invention there is provided a device for the dispensing of powder, the device being of the type in which a generated gas flow entrains the powder to be dispensed and carries the powder from the device via a barrel, the barrel having a bore including a main portion with a continuous internal surface, characterised in that
(a) the length of the main portion is at least fifteen times its maximum internal diameter; and
(b) the barrel has an outwardly flared outlet portion; and/or
(c) the internal bore of the main portion is tapered.

The device according to the invention is advantageous in that the form of the barrel may lead to certain improvements in performance. In particular, the form of the barrel may be chosen in such a way that the shape of the plume of powder emitted from the device is optimised for a particular application. Thus, the distribution of powder on the intended site of application may be made more uniform. In addition, the velocity of the powder plume may be increased, and this may result in the form of the plume being maintained over greater distances, so that the powder plume remains intact when projected over larger distances than those obtained with a conventional device, enabling the powder delivery device to be held a greater distance from the desired delivery site. This may be advantageous, for instance, in surgical procedures where it may not be possible to hold the device close to the intended site of application of the powder. In addition, fall-off of powder from the plume, under the influence of gravity, may be reduced, leading to reduced deposition of powder at undesired locations. In addition, the outlet may be less prone to clogging, improving performance and reducing the maintenance required. Dispersion of the powder as it leaves the device may also be improved. In general, the device according to the invention may operate effectively over a wide range of gasflow pressures, eg from about 0.5bar to about 7bar, though for most applications pressures in the range 0.5bar to 2bar are generally satisfactory, eg from 0.5bar to 1.5bar.

In the device of the invention, the length of the main portion is at least fifteen times its maximum internal diameter; and the internal bore of the main portion is tapered.

In the device of the invention, the barrel has an outwardly flared outlet portion.

The barrel may be rigid or it may be flexible.

In rigid embodiments, the length of the main portion of the barrel is at least fifteen times, eg at least eighteen times or at least twenty times, its maximum internal diameter, or more.

In rigid embodiments, the length of the main portion is typically between about 30mm and 100mm, eg about 50mm or about 75mm. The maximum internal diameter of the main portion is typically from 1 mm to 6mm, eg about 2mm or about 3mm.

In flexible embodiments of the barrel, the barrel is generally longer than for rigid embodiments, the length of the main portion of the barrel typically being between about 50mm and about 300mm, more commonly between about 50mm and about 200mm, and typically between about 100mm and 180mm, eg about 150mm. The internal diameter of the barrel in such embodiments is most commonly between about 1 mm and about 6mm, typically between about 1.5mm and about 4mm, eg about 2mm or about 3mm.

In order for the barrel to be flexible, its main portion is typically formed as a plastics tube, the plastics material being such that the tube may be manually deformed to the required shape without occlusion of the internal bore. The nature of the required shape will generally be dictated by the circumstances of the procedure in which the device is being used. Most commonly, a single bend may be introduced into the barrel by the user of the device, but in other situations it may be necessary to form the barrel into a more complex shape, eg with a double bend (ie an "S"-shape or the like). It has surprisingly been found that, even when bent into shapes as extreme as are ever likely to be required in practice (eg two 90° bends), the pattern of powder dispensed from the device can remain satisfactory in terms of the geometry of the plume of powder emerging from the distal end of the barrel and the pattern of deposition of the powder.

The nature of the plastics material from which the flexible elongate barrel is formed may be such that the tube retains the shape into which it is deformed, at least for as long as is required during normal use. Preferably, however, the barrel incorporates one or more malleable elongate members that can be readily deformed, and which have the effect of retaining the barrel in the desired configuration until it is once again manually deformed to another configuration. Such members may take the form of wires or rods of metal or other malleable material that are incorporated into the barrel. Most commonly, such wires or rods are disposed parallel to the longitudinal axis of the barrel. In some embodiments, the main portion of the barrel is a tube having more than one lumen, one lumen (normally the one having the greatest cross-sectional area) constituting the bore of the barrel along which powder is dispensed, and at least one lumen accommodating one or more wires or rods. In other embodiments, one or more wires or rods are embedded in the plastics material from which the barrel is formed. There may be just one such wire or rod, or there may be two, three, four or more such wires. Where there are more than one wire or rod, those wires or rods are most commonly equiangularly spaced around the main portion of the barrel.

Forms of material that are suitable for the malleable wires or rods are copper wire and copper-coated steel wire. In one currently preferred embodiment, a single such wire is embedded in the wall of the tube that constitutes the main portion of the barrel. In other embodiments, two, three or four such wires may be used.

In other embodiments, another form of malleable material may be used to enable the form of the barrel to be altered. For instance, the main portion of the barrel may be provided, along the whole or part of its length, with a sheath of material that holds its shape when deformed, eg a foam material of the type known as memory foam or visco-elastic foam..

In embodiments in which the internal bore of the main portion is tapered, the internal diameter of the bore may decrease from the upstream to the downstream end of the main portion, ie the internal bore of the main portion may converge. Alternatively, the internal diameter of the bore may increase from the upstream to the downstream end of the main portion, ie the internal bore of the main portion may diverge. The taper angle may typically be in the range 0.5° to 3°, more commonly 0.5° to 2°.

Embodiments in which the main portion of the barrel is flexible typically have a main portion that is considerably longer than is the case for rigid embodiments. Those flexible embodiments are typically formed by extrusion and in such cases will therefore have an internal bore of constant cross-section. Embodiments in which the main portion of the barrel is rigid are more commonly formed by injection moulding, in which case the internal bore of the main portion of the barrel may have a uniform cross-section or may, more preferably, be tapered.

The flared outlet outlet portion typically has a length of between 5mm and 25mm, more commonly between 5mm and 10mm. The internal diameter of the outlet portion may increase from its upstream to its downstream end by a factor of two or more. As will be readily appreciated, the term "flared" refers to the internal shape of the outlet, ie to a widening of the outlet from its upstream to its downstream end. That widening may or may not be reflected in the external shape of the outlet.

Briefly summarised, the device disclosed in WO 2010/070333 has a main body that may comprise upper and lower housing components formed in plastics material by injection moulding. The main body may have the general form of an elongate cylinder that is adapted to be held in a user's hand, the underside of the lower component being shaped to facilitate such grip. A push button-type actuator may be mounted in the top of the main body such that, when the device is held by the user, the actuator can be depressed by the thumb of the hand that holds the device. A flexible tube may extend from the rear end of the device and may be adapted to be connected to a gas source, eg a source of compressed air. A connector may be provided at the distal end of the tube. A vial containing the powder that is to be dispensed from the device may be coupled to the device, eg via an upstanding spigot that is received within the mouth of the vial. The barrel may extend from the front end of the device.

The barrel may be provided with a mounting that enables its orientation relative to the main body of the device of which it forms part to be varied. For instance, the barrel may engage the main body of the device in the manner of a ball-and-socket connector, so that the orientation of the barrel may be adjusted, eg manually by the operator.

In presently preferred embodiments, however, the barrel is connected to the main body of the device in a fixed orientation. The barrel may be connected to the main body by a threaded connection, or by a suitable quick release connection such as a bayonet fitting. Other forms of connection may alternatively be used, eg a luer lock-type connection, or an interference fit or the like.

The device according to the invention may be manufactured using medical grade materials, most components being most conveniently manufactured in plastics by techniques such as injection moulding and extrusion. Where appropriate, components may be manufactured in other materials, eg glass or metal.

The device according to the invention may be used to deliver a wide variety of powders to a surface of the body. Such powders include agents intended to have a therapeutic effect, either in terms of a pharmacological effect on the body or as disinfectants or the like useful in the prevention or treatment of infections. One particular field in which the device of the invention is useful, however, is for the delivery of haemostatic powder compositions to internal tissues exposed during surgical procedures or after traumatic injury. Such haemostatic compositions, which may also be described as tissue sealants, may for instance comprise dry powder mixtures of fibrinogen and thrombin. Such a mixture is essentially inert when formulated in the dry state, but once hydrated, eg upon application to a bleeding wound, the mixture leads to the production of fibrin which cross-links to form a blood clot.

It is also disclosed a method of delivering a haemostatic composition to an internal tissue exposed during surgical procedures or after traumatic injury, which method comprises providing a device as described above, which device is charged with a quantity of a haemostatic composition in dry powder form, and dispensing said composition from said device onto said tissue.

The invention will now be described in greater detail, by way of illustration only, with reference to the accompanying drawings, in which
Figure 1 is a perspective view of a first embodiment of a powder delivery device according to the invention;
Figure 2 is a side view of the device of Figure 1;
Figure 3 shows a barrel that forms part of the device of Figure 1;
Figure 4 is a cross-sectional view of the barrel of Figure 3;
Figures 5(a) and (b) illustrate the range of vertical movement of the barrel;
Figures 6(a) and 6(b) illustrate the range of lateral movement of the barrel;
Figure 7 is a perspective view of a second embodiment of a powder delivery device according to the invention, showing the barrel separated from the main body of the device;
Figure 8 is a perspective view from behind of the barrel of the device of Figure 7;
Figure 9 is a cross-sectional view of the barrel of Figure 8;
Figure 10 is a perspective view of a third embodiment of a powder delivery device according to the invention, comprising the main body of the second embodiment with an alternative form of barrel attached to it;
Figure 11 is a perspective view of the barrel shown in Figure 10; and
Figure 12 is a cross-sectional view of the barrel of Figure 11.

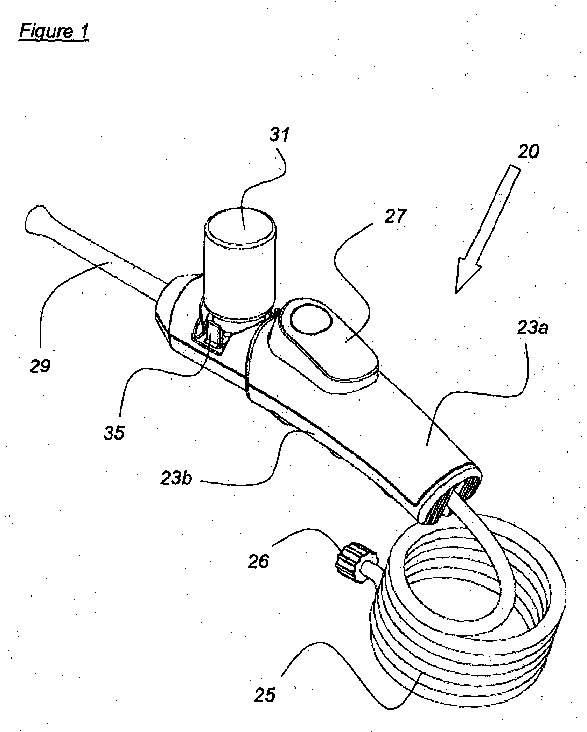

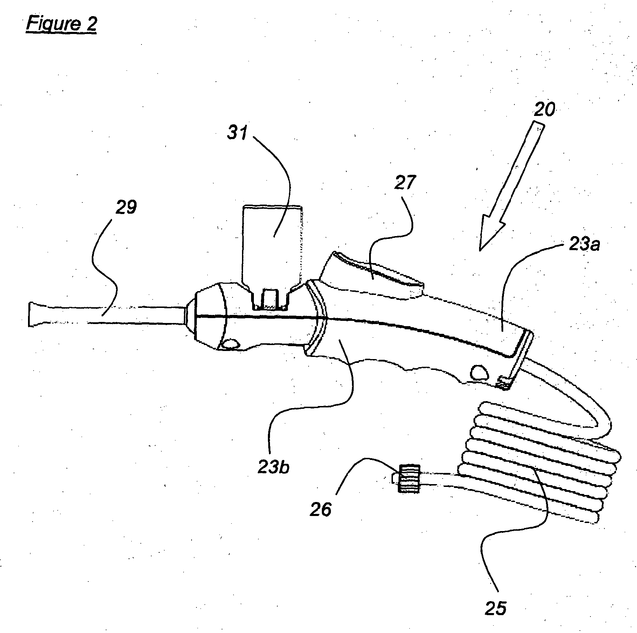

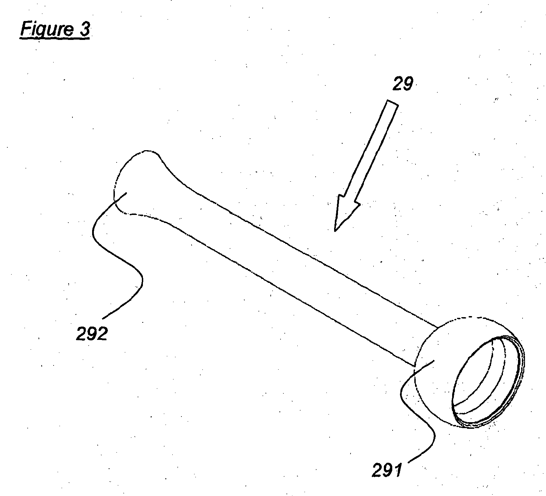

Referring first to Figures 1 and 2, a first embodiment of a powder delivery device in accordance with the invention is generally designated 20. Save for the inventive modification described in greater detail below, the device 20 is of similar form to the device illustrated in Figures 3 to 12 of WO2010/070333. Briefly summarised, the device 20 has a main body that comprises upper and lower housing components 23a,23b that are formed in plastics material by injection moulding. The main body has the general form of an elongate cylinder that is adapted to be held in a user's hand, the underside of the lower component 23b being shaped to facilitate such grip. A push button-type actuator 27 is mounted in the top of the main body such that, when the device 20 is held by the user, the actuator 27 can be depressed by the thumb of the hand that holds the device 20.

A flexible tube 25 extends from the rear end of the device 20 and is adapted to be connected to a gas source, eg a source of compressed air (not shown). A suitable connector 26 is provided at the distal end of the tube 25.

A glass vial 31 is coupled to the device 20 via an upstanding spigot (not visible) that is received within the mouth of the vial 31. A pair of clips 35 engage with a peripheral lip of the vial 31, so as to hold it securely in place. In other embodiments, the vial 31 may simply engage the spigot or similar formation with an interference fit and/or the spigot or similar formation may have a degree of resilience enabling a clip-type fitting to the vial. To facilitate sealing engagement of the vial 31 with the device 20, the formation that receives the mouth of the vial 31 may incorporate or be formed from a suitable elastomeric material (eg a thermoplastic elastomer). A device 20 having such a fitting may be produced using a two-shot moulding process.

As for the device described in WO010/070333, the front end of the device 20 is provided with a tubular barrel 29, through which powder is dispensed from the device 20. The barrel 29 in the present invention, however, is rather different in form to that described in the earlier patent application, as is discussed in greater detail below.

The base of the spigot with which the vial 31 is engaged is closed by a perforated plate, such that when the device is in the operative orientation shown in Figures 1 and 2, powder contained within the vial 31 rests upon the perforated plate. When the actuator 27 is depressed, gas is caused to flow through the device 20, as described in WO2010/070333. The flow of gas has two, related,.effects. First, the gas flow drives a mechanical agitator in the form of a ball that is held captive within a circular track; rotation of the ball around the track induces vibrations in the device 20, the effect of which is to dislodge powder from the vial 31, through the perforated plate that constitutes the base of the spigot that is engaged by the vial 31. Secondly, a proportion of the the gas flow is directed at the underside of the perforated plate, entrains the powder that is dislodged from the vial 31 and carries it from the device 20 via the barrel 29.

Thus, to dispense powder from the device 20, the user holds the device 20 in one hand, directs the barrel 29 at the intended site of application of the powder, and depresses the actuator 27 with the thumb. This permits gas to flow through the device 20, causing the ball to rotate rapidly around the track and inducing a degree of mechanical vibration that is transmitted to the vial 31. Most of the gasflow is vented from the device 20. However, a small proportion of gas is directed at the underside of the perforated plate. The mechanical agitation of the device 20 caused by rotation of the ball within the track facilitates the release of powder from the vial 31. The powder is entrained in the flow of air that escapes from the device 20 via the barrel 29. The powder is blown out of the device 1 and deposited on the site of application.

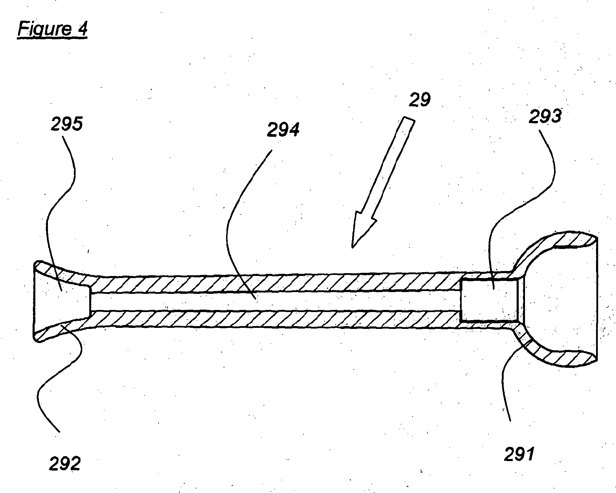

As noted above, the device 20 differs from that disclosed in WO2010/070333 in the form of the barrel 29. The barrel 29 is shown in Figures 3 and 4.

As can be seen in Figure 3, the barrel 29 is elongate and of circular cross-section, extending from a ball-type connector 291 at one end to a flared outlet 292 at the other. The ball-type connector 291 is captivated within an opening at the front of the main body of the device 20, between the upper and lower housing components 23a,23b, in the manner of a ball-and-socket joint.

As can be seen from Figure 4, the barrel 29 has an internal bore having an entry portion 293 that is of constant diameter, a main bore 294 that is of reduced diameter relative to the entry portion 292, and an outlet portion 295 within the flared outlet 292. The diameter of the main bore 294 reduces along its length.

The overall length of the barrel 29 is approximately 70mm. The entry portion 293 of the internal bore has a diameter of approximately 5mm and a length of approximately 8mm. The diameter of the main bore 294, which has a length of approximately 48mm, reduces from approximately 3.5mm adjacent the entry portion 293 to approximately 2mm where the main bore 294 meets the outlet portion 295. The angle of convergence of the internal wall of the main bore 294 relative to its longitudinal axis is thus approximately 1°.

The entry portion 293 receives a tube (not visible in the drawings) by which the gasflow and entrained powder are fed from the main body of the device 20 to the barrel 29.

The narrowing of the main bore 294 may cause an increase in velocity of the gasflow as it exits the device 20 and, together with the flared form of the outlet portion 295, this may help to maintain the shape of the powder plume emitted from the device 20, resulting in good coverage of the application site with powder. Fall off of powder from the emitted plume, under the influence of gravity, may be reduced, minimising the amount of powder that is dispensed onto sites other that to which the powder is intended to be delivered. The flared form of the outlet portion 295 also leads to a reduced likelihood of clogging.

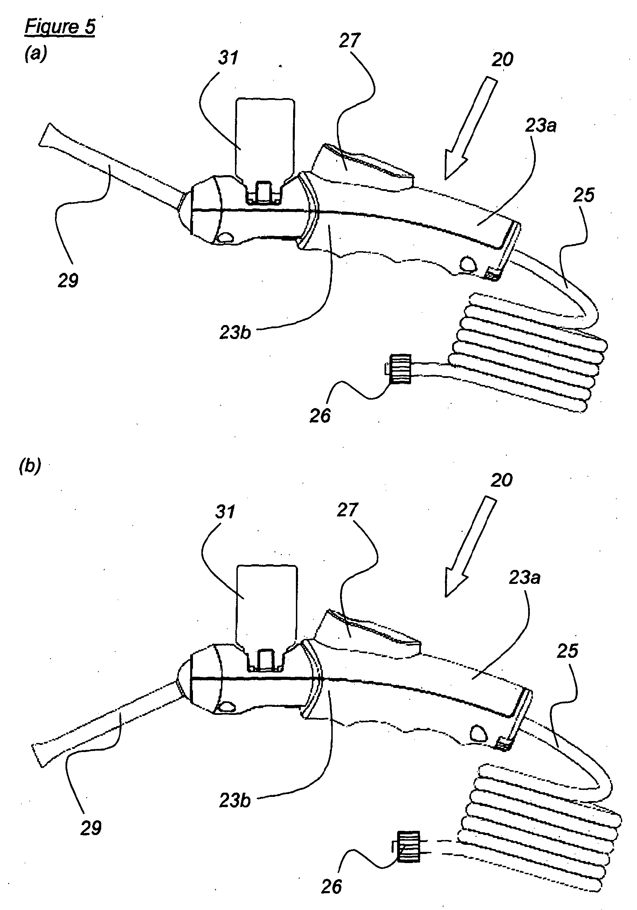

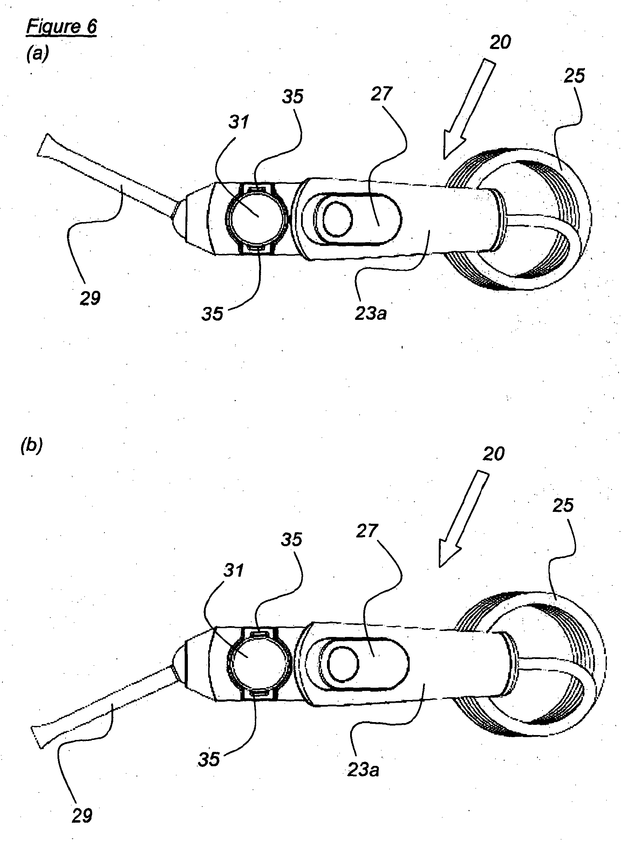

In this embodiment, directional control over the emitted powder is achieved by virtue of the fact that the ball-type connector 291 permits the orientation of the barrel 290 relative to the main body of the device 20 to be varied over a wide range, both vertically (see Figures 5(a) and 5(b)) and laterally (Figures 6(a) and 6(b)).

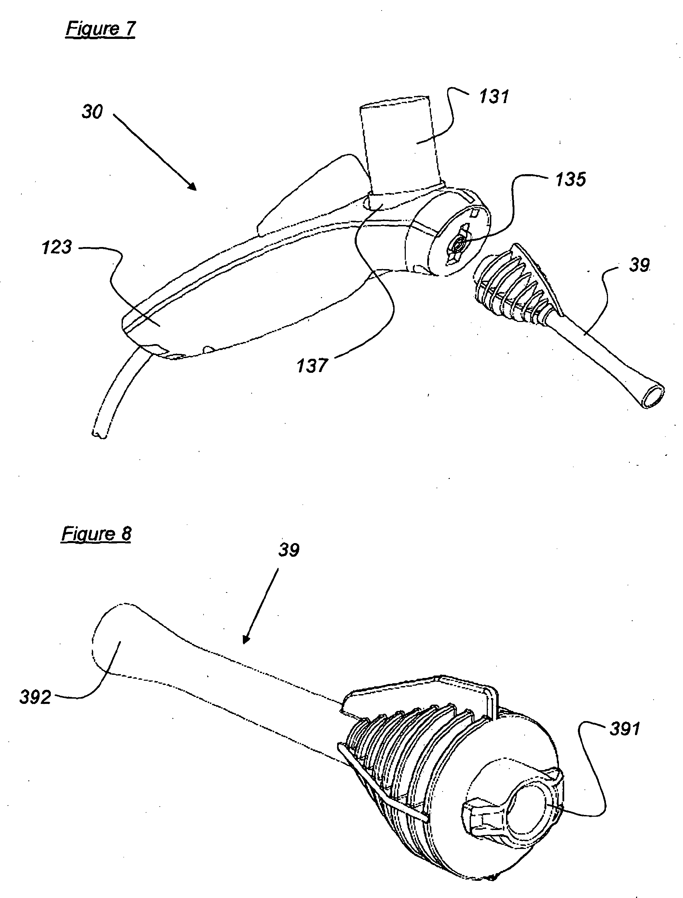

Turning now to Figure 7, a second embodiment of a powder delivery device according to the invention is generally designated 30. The device 30 is broadly similar to the first embodiment 20 described above in that it comprises a main body 123 that receives vial 131 of powder. In this embodiment, the vial 131 is received within a ring 137 of thermoplastic elastomer that is moulded onto the upper component of the main body 123 in a two-shot moulding process.

Like the first embodiment 20, the device 30 comprises a tubular outlet barrel 39. In the second embodiment 30, however, the barrel 39 connects to the main body 123 by means of a bayonet fitting and has a fixed orientation relative to the main body 123.

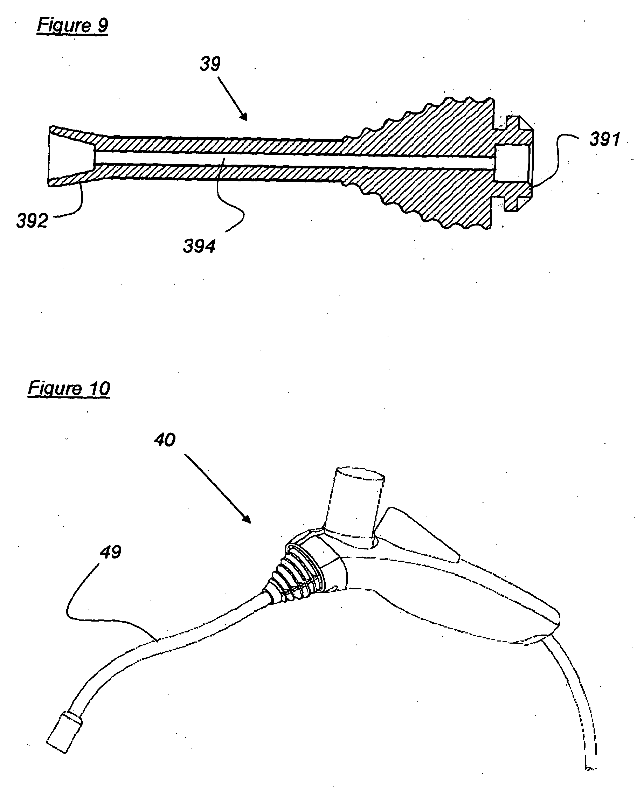

As can be seen in Figure 8, the face of the barrel that abuts the main body 123 is formed with a male bayonet spigot 391 with a central recess that receives the open end of a tube 135 (see Figure 7) by which the gasflow and entrained powder are fed from the main body 123 of the device to the barrel 39. The barrel 39 is injection moulded in rigid plastics material (eg ABS) and (referring to Figure 9), like .the barrel 29 of the first embodiment 20, is formed with an internal bore 394 that diminishes along its length (in this case from an internal diameter of approximately 2.9mm to an internal diameter of approximately 2mm). The barrel 39 has a flared outlet 392.

As for the first embodiment 20, the narrowing of the main bore 394 may cause an increase in velocity of the gasflow as it exits the device 30 and, together with the flared form of the outlet 392, this may help to maintain the shape of the powder plume emitted from the device 30, resulting in good coverage of the application site with powder. Fall off of powder from the emitted plume, under the influence of gravity, may be reduced, minimising the amount of powder that is dispensed onto sites other that to which the powder is intended to be delivered. The flared form of the outlet 392 also leads to a reduced likelihood of clogging.

Finally, Figure 10 depicts a third embodiment of a powder delivery device according to the invention, which is generally designated 40. This embodiment differs from the second embodiment 30 solely in the form of the outlet barrel 49. Indeed; the outlet barrel 39 of the second embodiment 30 and the outlet barrel 49 of the third embodiment 40 are interchangeable.

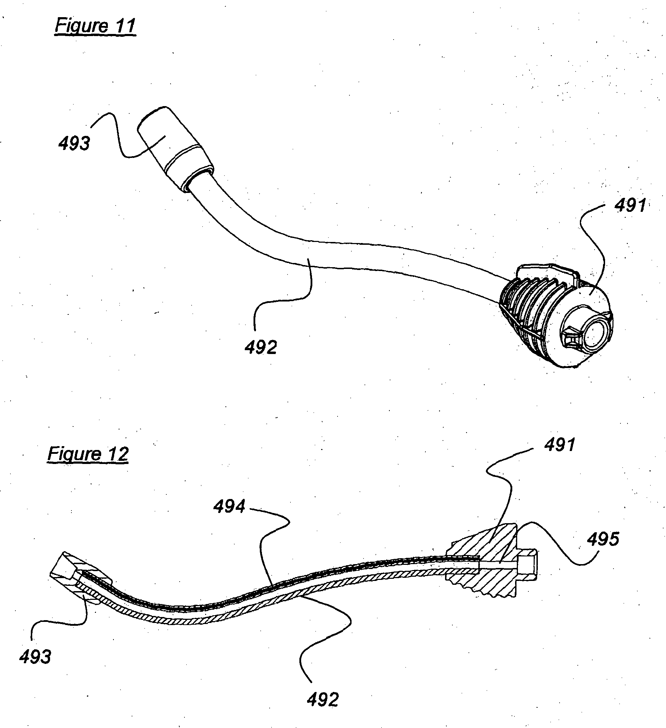

The form of the barrel 49 is shown in greater detail in Figures 11 and 12. The barrel 49 comprises three components: a bayonet hub 491, a flexible plastics tube 492 and an outlet tip 493.

The bayonet hub 491 is injection moulded in rigid plastics material (eg polyamide) and corresponds in overall shape to the proximal end of the rigid barrel 39 of the second embodiment 30. It has a central bore 495 that terminates in a socket within which one end of the flexible tube 492 is received. That end of the tube 492 may be fixed to the hub 491 by adhesive, by ultrasonic welding or by any other suitable means. The other end of the tube 492 is received within a similar socket formed in the outlet tip 493 (which is also formed by injection moulding of a suitable material, eg polyamide) and may be fixed by similar means. The distal (downstream) part of the outlet tip 493 has a flared internal bore.

The flexible tube 492 is formed by extrusion in polyurethane, a copper-coated steel wire 494 being embedded in the wall of the flexible tube 492 during manufacture. A user of the device may therefore form the flexible tube 492 into a desired configuration, the effect of the wire 494 being to retain the tube 492 in that configuration during use. The third embodiment 40 may be particularly useful in surgical procedures in which it is desired to deposit the powder onto surfaces that are hidden and at which a straight barrel cannot be directed.

## Claims

1. A device for the dispensing of powder, the device being of the type in which a generated gas flow entrains the powder to be dispensed and carries the powder from the device via a barrel (29;39;49), the barrel (29;39;49) having a bore including a main portion (294;394) with a continuous internal surface,
(a) the length of the main portion (294;394) is at least fifteen times its maximum internal diameter; **characterised in that**
(b) the barrel (29;39;49) has an outwardly flared outlet portion (292;392;493).

2. A device as claimed in Claim 1, wherein the internal bore of the main portion (294;394) is tapered.

3. A device as claimed in any preceding claim, wherein the barrel (29;39) is rigid.

4. A device as claimed in Claim 3, wherein the length of the main portion (294;394) is between 30mm and 100mm, and the maximum internal diameter of the main portion (294;394) is from 1mm to 6mm.

5. A device as claimed in any one of Claims 1 to 2, wherein the barrel (49) is flexible.

6. A device as claimed in Claim 5, wherein the main portion of the barrel (49) has a length of between 50mm and 300mm, and the internal diameter of the barrel (49) is between 1mm and 5mm.

7. A device as claimed in any one of Claims 5 to 6, wherein the main portion of the barrel (49) comprises a plastics tube.

8. A device as claimed in Claim 5, wherein the barrel (49) incorporates one or more malleable elongate wires or rods (494).

9. A device as claimed in Claim 8, wherein the wires or rods (494) are embedded in the plastics material from which the barrel (49) is formed.

10. A device as claimed in Claim 8 or Claim 9, wherein the wires or rods (494) are one or more copper or copper-coated steel wires.

11. A device as claimed in any preceding claim, wherein the internal diameter of the main portion (294;394) decreases from the upstream to the downstream end of the main portion, at a taper angle of 0.5° to 3°.

12. A device as claimed in Claim 1, wherein the outlet portion (292;392;493) has a length of between 5mm and 25mm, and the internal diameter of the outlet portion (292;392;493) increases from its upstream to its downstream end by a factor of two or more.

13. A device as claimed in any preceding claim, wherein the barrel (29) is provided with a mounting that enables its orientation relative to the main body of the device of which it forms part to be varied.

14. A device as claimed in Claim 13, wherein the mounting is a ball-and-socket connector.

15. A device as claimed in any one of Claims 1 to 12, wherein the barrel (39) is connected to the main body of the device in a fixed orientation, by a bayonet fitting.

## Patentansprüche

1. Eine Vorrichtung zum Abgeben von Pulver, wobei die Vorrichtung von dem Typ ist, bei dem eine erzeugte Gasströmung das abzugebende Pulver mitführt und das Pulver von der Vorrichtung über eine Hülse (29; 39; 49) befördert, wobei die Hülse (29; 39; 49) eine Bohrung mit einem Hauptabschnitt (294; 394) mit einer kontinuierlichen Innenfläche hat,
(a) wobei die Länge des Hauptabschnitt (294; 394) zumindest das Fünfzehnfache seines maximalen Innendurchmessers ist,
**dadurch gekennzeichnet, dass**
(b) die Hülse (29; 39; 49) einen nach außen aufgeweiteten Auslassabschnitt (292; 392; 493) hat.

2. Vorrichtung nach Anspruch 1, wobei die Innenbohrung des Hauptabschnitts (294; 394) konisch ist.

3. Vorrichtung nach einem der vorhergehenden Ansprüche, wobei die Hülse (29; 39) starr ist.

4. Vorrichtung nach Anspruch 3, wobei die Länge des Hauptabschnitts (294; 394) zwischen 30 mm und 100 mm ist und der maximale Innendurchmesser des Hauptabschnitts (294; 394) von 1 mm bis 6 mm ist.

5. Vorrichtung nach einem der Ansprüche 1 bis 2, wobei die Hülse (49) flexibel ist.

6. Vorrichtung nach Anspruch 5, wobei der Hauptabschnitt der Hülse (49) eine Länge zwischen 50 mm und 300 mm hat und der Innendurchmesser der Hülse (49) zwischen 1 mm und 5 mm ist.

7. Vorrichtung nach einem der Ansprüche 5 bis 6, wobei der Hauptabschnitt der Hülse (49) ein Plastikrohr aufweist.

8. Vorrichtung nach Anspruch 5, wobei die Hülse (49) einen unter Druck verformbaren, langgestreckten Draht oder Stab (494) oder mehrere davon aufweist.

9. Vorrichtung nach Anspruch 8, wobei die Drähte oder Stäbe (494) in das Plastikmaterial eingebettet sind, aus dem die Hülse (49) geformt ist.

10. Vorrichtung nach Anspruch 8 oder 9, wobei die Drähte oder Stäbe (494) ein Kupferdraht oder ein kupferbeschichteter Stahldraht oder mehrere davon sind.

11. Vorrichtung nach einem der vorhergehenden Ansprüche, wobei der Innendurchmesser des Hauptabschnitts (294; 394) sich von dem Stromauf- zu dem Stromab-Ende des Hauptabschnitts mit einem Kegelwinkel von 0,5° bis 3° verringert.

12. Vorrichtung nach Anspruch 1, wobei der Auslassabschnitt (292; 392; 493) eine Länge von zwischen 5 mm und 25 mm hat und der Innendurchmesser des Auslassabschnitts (292; 392; 493) sich von seinem Stromauf- zu seinem Stromab-Ende um einen Faktor von zwei oder mehr erhöht.

13. Vorrichtung nach einem der vorhergehenden Ansprüche, wobei die Hülse (29) mit einer Befestigung versehen ist, die ermöglicht, dass sich ihre Ausrichtung in Bezug auf den Hauptkörper der Vorrichtung, von dem diese einen Teil bildet, ändert.

14. Vorrichtung nach Anspruch 13, wobei die Befestigung ein Kugel-Pfanne-Verbinder ist.

15. Verbinder nach einem der Ansprüche 1 bis 12, wobei die Hülse (39) mit dem Hauptkörper der Vorrichtung in einer festgelegten Ausrichtung durch ein Bajonett-Anschlussstück verbunden ist.

## Revendications

1. Dispositif de distribution d'une poudre, le dispositif étant du type dans lequel un flux de gaz généré entraîne la poudre à distribuer et achemine la poudre depuis le dispositif à l'aide d'un barillet (29 ; 39 ; 49), le barillet (29 ; 39 ; 49) ayant un alésage qui comprend une partie principale (294 ; 394) avec une surface interne continue,
(a) la longueur de la partie principale (294 ; 394) étant au moins égale à quinze fois son diamètre interne maximal ; **caractérisé en ce que**
(b) le barillet (29 ; 39 ; 49) possède une partie de sortie évasée vers l'extérieur (292 ; 392 ; 493).

2. Dispositif selon la revendication 1, dans lequel l'alésage intérieur de la partie principale (294 ; 394) est effilé.

3. Dispositif selon l'une quelconque des revendications précédentes, dans lequel le barillet (29 ; 39) est rigide.

4. Dispositif selon la revendication 3, dans lequel la longueur de la partie principale (294 ; 394) se situe entre 30 mm et 100 mm, et le diamètre interne maximal de la partie principale (294 ; 394) est compris entre 1 mm et 6 mm.

5. Dispositif selon l'une quelconque des revendications 1 à 2, dans lequel le barillet (49) est flexible.

6. Dispositif selon la revendication 5, dans lequel la partie principale du barillet (49) possède une longueur comprise entre 50 mm et 300 mm, et le diamètre interne du barillet (49) est compris entre 1 mm et 5 mm.

7. Dispositif selon l'une quelconque des revendications 5 à 6, dans lequel la partie principale du barillet (49) comprend un tube en plastique.

8. Dispositif selon la revendication 5, dans lequel le barillet (49) comprend un(e) ou plusieurs fil(s) ou tige(s) allongé(es) malléable(s) (494).

9. Dispositif selon la revendication 8, dans lequel les fils ou les tiges (494) sont intégré(e)s au matériau en plastique dont le barillet (49) est formé.

10. Dispositif selon la revendication 8 ou 9, dans lequel les fils ou les tiges (494) sont un ou plusieurs fil(s) en cuivre ou en acier recouvert de cuivre.

11. Dispositif selon l'une quelconque des revendications précédentes, dans lequel le diamètre interne de la partie principale (294 ; 394) diminue entre l'extrémité amont et l'extrémité aval de la partie principale, à un angle d'effilement de 0,5 à 3°.

12. Dispositif selon la revendication 1, dans lequel la partie de sortie (292 ; 392 ; 493) possède une longueur comprise entre 5 mm et 25 mm, et le diamètre interne de la partie de sortie (292 ; 392 ; 493) diminue entre son extrémité amont et son extrémité aval selon un facteur de deux ou plus.

13. Dispositif selon l'une quelconque des revendications précédentes, dans lequel le barillet (29) est muni d'un support qui permet de modifier son orientation par rapport au corps principal du dispositif dont il fait partie.

14. Dispositif selon la revendication 13, dans lequel le support est un connecteur à rotule.

15. Dispositif selon l'une quelconque des revendications 1 à 12, dans lequel le barillet (39) est relié au corps principal du dispositif selon une orientation fixe, par une fixation à baïonnette.
